# EUROPÄISCHE PATENTSCHRIFT

(11) **EP 0 475 889 B1**
(45) Veröffentlichungstag und Bekanntmachung des Hinweises auf die Patenterteilung: **03.05.1995**
(21) Anmeldenummer: 91810613.9
(22) Anmeldetag: 06.08.1991
(51) Int. Cl.: A61F 2/08

(54) **Als Bänder- oder Sehnenersatz dienendes Implantat**
Implant serving as ligament or tendon
Implant servant de ligament ou tendon

(30) Priorität: 07.09.1990 CH 2910/90
(43) Veröffentlichungstag der Anmeldung: 18.03.1992
(73) Patentinhaber: SULZER Medizinaltechnik AG, CH-8401 Winterthur (CH); PROTEK AG, 3110 Münsingen-Bern (CH)
(72) Erfinder: Flückiger, Hans, CH-8618 Oetwil am See (CH); Freudiger, Stefan, CH-3047 Bremgarten (CH); Koch, Rudolf, CH-8267 Berlingen (CH)
(74) Vertreter: Trieblnig, Adolf

(56) Entgegenhaltungen:
- EP-A- 0 145 492
- FR-A- 2 395 012
- US-A- 3 176 316
- US-A- 4 584 722
- US-A- 4 883 486

## Beschreibung

Die Erfindung betrifft ein als Bänder- oder Sehnenersatz dienendes Implantat aus einer Anzahl von in Längsrichtung verlaufenden Fadensträngen, die durch Zwirnen, Flechten oder Seilen in ihrer Festigkeit verstärkt sind, oder von monofilen Längsfäden, wobei die Gesamtheit der Fadenstränge oder Fäden an einem Ende auf einem Teil ihrer Länge von einem textilen Strumpf zusammengehalten ist.

Ein Implantat der vorstehend genannten Art ist beispielsweise aus der FR-A-2 395 012 bekannt. Müssen derartige Implantate durch Knochenbohrungen - beispielsweise als Kreuzbandersatz im Femur und/oder in der Tibia - hindurchgeführt werden, so ergeben sich dabei häufig Schwierigkeiten besonders, wenn das durch den Knochen zu ziehende Ende des Implantates in einzelne Stränge oder Fäden aufgelöst oder als Verankerungsöse ausgebildet ist.

Eine weitere Ausführungsform ist in der EP-A-0 145 492 gezeigt. Diese handelt von einem im implantierten Zustand ummantelten künstlichen Band, dessen Mantel in einem Mittelstück entweder längsgeschlitzt ist oder im wesentlichen nur längsorientierte Fasern aufweist, um Ermüdungsbrüchen bei Biegebelastung des Bandes vorzubeugen. Dabei ist der Mantel am Bandende an der Befestigung des Bandes - zum Beispiel in Oesenform - mitbeteiligt. Dies hat den Nachteil, dass die Materialeigenschaften des Mantelmaterials bei der Befestigung des Bandes mitberücksichtigt werden müssen.

Aufgabe der Erfindung ist es, hier Abhilfe zu schaffen und dem Operateur das Durchziehen eines Implantates der vorstehend genannten Art durch Knochenbohrungen zu erleichtern.

Diese Aufgabe wird mit den kennzeichnenden Merkmalen des unabhängigen Anspruches 1 gelöst.

Im Gegensatz zur EP-A-0 145 492 besteht der Anfang des Strumpfes aus einem schlauchförmigen Teilstück, welches von den Fadensträngen seitlich weggedrückt wird, weil diese durch eine Oeffnung in der Mantelfläche in den Strumpf hineinragen und seine Richtung bestimmen. Dabei werden durch ein Anfangsteilstück in Schlauchform einzelne seitlich abstehende Fäden oder Fasern als Teilstücke beim Einziehen ausgeschlossen.

Bei der Implantation wird das Implantat, mit dem von dem Strumpf umhüllten Ende voran, in die Knochenbohrung eingefädelt und durch sie hindurch gezogen. Der Strumpf umhüllt das Implantat dabei nur soweit, dass nach dem Durchziehen sein keine Fadenstränge umhüllendes Anfangsteilstück am Ende der Knochenbohrung wieder "austritt". Dieses Ende wird dann abgeschnitten, wodurch sich das textile Muster des Strumpfes zunächst teilweise auflöst oder, beispielsweise bei einem Gestrick, aufgezogen werden kann. Damit ist der Reststrumpf auf einfache Weise von dem durchgezogenen Implantat zu entfernen.

Einfädeln und Durchziehen können erleichtert werden, wenn sein vom Strumpf umhülltes Ende sich durch unterschiedliche Längen der einzelnen Fadenstränge oder Fäden konisch verjüngt.

Weiterhin hat es sich als vorteilhaft erwiesen, wenn der Strumpf aus einem Geflecht besteht, da durch Einstellen des Flechtwinkels - d.h. des Winkels, den die geflochteten Fäden mit der Längsachse des Geflechts bilden - die Kompaktheit und Steifigkeit des umflochtenen Implantatendes in gewissem Umfang variiert werden kann; dadurch wird zum einen der Durchmesser der umgeflochteten Fadenstränge reduziert und zum anderen die "Haftung" des Strumpfes auf dem Implantatende durch Reibung gewährleistet.

Im folgenden wird die Erfindung anhand von Ausführungsbeispielen im Zusammenhang mit der Zeichnung näher erläutert.
- Fig. 1: zeigt schematisch das neue Implantat mit einem umflochteten Ende;
- Fig. 2: gibt das Implantat nach Fig. 1 als in ein Kniegelenk eingesetztes Kreuzband nach dem Durchziehen durch Femur und Tibia wieder;
- Fig. 3 und 4: verdeutlichen schematisch die Herstellung einer anderen Ausführungsform des Implantates.

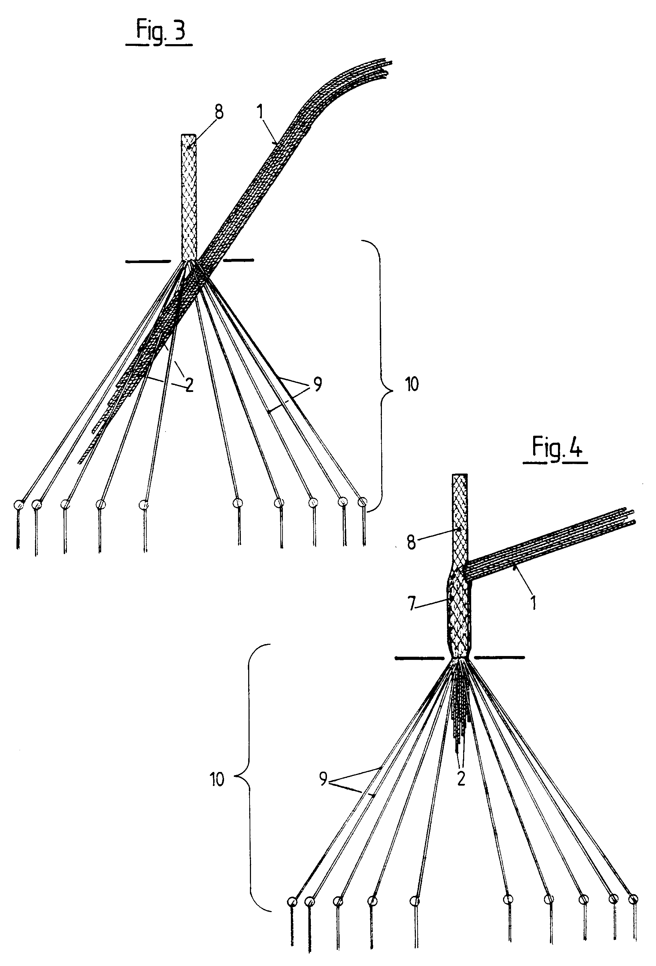

Das Implantat 1, das als Kreuzbandersatz verwendet wird, besteht mindestens zum Teil aus in Längsrichtung verlaufenden monofilen Fäden oder aus Fadensträngen 2; die einzelnen Fadenstränge 2 sind dabei durch Flechten, Zwirnen oder Seilen in ihrer Festigkeit verstärkt worden. Weiterhin ist das eine Ende des Implantats 1 als Oese 3 für den Durchtritt einer Verankerungsschraube ausgebildet, die in die Tibia 4 (Fig. 2) eingeschraubt wird.

Wie Fig. 2 erkennen lässt, muss das Implantat, das mit einem Ende am Femur 5 fixiert wird - mit dem als Oese 3 ausgebildeten Ende voran - durch Bohrungen 6 im Femur 5 und in der Tibia 4 gezogen werden. Gemäss der vorliegenden Erfindung ist dieses Ende daher von einem geflochtenen Strumpf 7 umschlossen. Dieser weist, ehe er das Implantat 1 umfasst, ein neben den Fadensträngen 2 liegendes Anfangsteilstück 8 auf, das noch keine Fadenstränge 2 umgreift und daher einen stark reduzierten Durchmesser hat.

Wie bereits beschrieben, wird das Teilstück 8 nach dem Durchziehen durch den oder die Knochen 5 und 4 von dem übrigen Strumpf 7 abgetrennt. Ist der Strumpf 7 geflochten, so fächern sich die einzelnen "vorgespannten" Fäden 9 (Fig. 3) des Geflechtes blumenkelchartig auf. Der Strumpf 7 kann dann - gegebenenfalls nach weiterer Lockerung seines Geflechtes durch den Operateur - von dem Ende des Implantates 1 abgezogen werden.

Besteht der Strumpf 7 jedoch aus einem einzigen endlosen Faden, so wird er einfach aufgezogen.

Die Herstellung des neuen Implantates 1 kann beispielsweise auf einer Flechtmaschine erfolgen; der Flechtbereich 10 einer solchen Maschine ist in Fig. 3 und 4 schematisch angedeutet. Auf einer solchen Maschine wird zunächst aus einzelnen Fäden 9, die als Mono- oder Multifilamente ausgebildet sein können, das Anfangsstück 8 geflochten. Hat dieses seine gewünschte Länge erreicht, so wird in den Flechtbereich 10 das mit einem Strumpf 7 zu ummantelnde Ende des Implantates eingebracht und das Flechten fortgesetzt bis der Strumpf 7 die Fadenstränge 2 des Implantates vollständig umschliesst oder gegebenenfalls darüberhinaus verlängert ist.

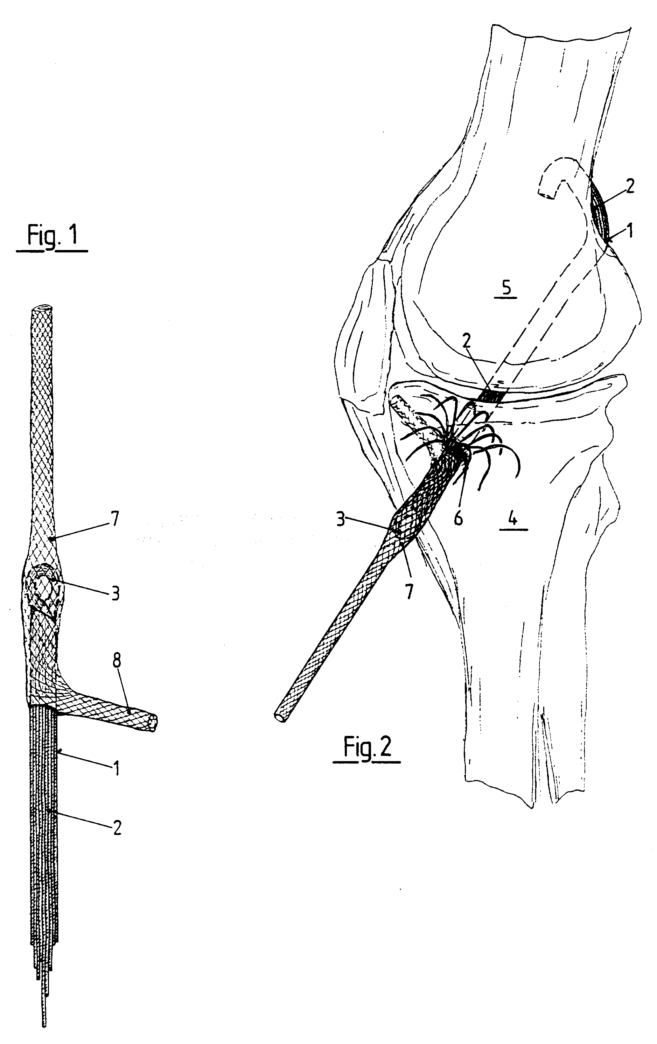

Im Gegensatz zu Fig. 1 und 2 besteht das Implantatende in Fig. 3 und 4 nicht aus einer Oese 3, sondern aus ungleich langen Fadensträngen 2; der Strumpf 7 erhält so eine konisch zulaufende Form, durch die das Einfädeln in und das Durchziehen durch eine Knochenbohrung 6 erleichtert werden.

## Patentansprüche

1. Als Bänder- oder Sehnenersatz dienendes Implantat (1) aus einer Anzahl von in Längsrichtung verlaufenden Fadensträngen (2), die durch Zwirnen, Flechten oder Seilen in ihrer Festigkeit verstärkt sind, oder von monofilen Längsfäden, wobei die Gesamtheit der Fadenstränge (2) oder Fäden an einem Ende auf einem Teil ihrer Länge von einem textilen Strumpf (7) zusammengehalten ist, dadurch gekennzeichnet, dass der aus einem Geflecht, Gestrick, Gewirk oder Gewebe bestehende Strumpf (7) an seinem über die Fadenstränge oder Fäden gezogenen Anfang zunächst ein die Fadenstränge (2) oder Fäden nicht umschliessendes, seitlich von den Fadensträngen oder Fäden abstehendes schlauchförmiges Teilstück (8) aufweist, das abtrennbar ist.

2. Implantat nach Anspruch 1, dadurch gekennzeichnet, dass sein vom Strumpf (7) umhülltes Ende sich durch unterschiedliche Längen der einzelnen Fadenstränge (2) oder Fäden konisch verjüngt.

3. Implantat nach Anspruch 1 oder 2, dadurch gekennzeichnet, dass der Strumpf (7) aus einem Geflecht besteht.

## Claims

1. An implant (1) serving as replacement for a ligament or tendon,
consisting of a number of strands (2) of thread which run in the longitudinal direction and are reinforced in their strength by twisting, braiding or warping, or of monofilar longitudinal threads, the whole of the strands (2) of thread or the threads being held together at one end over part of their length by a textile sock (7),
characterized in that the sock (7) consisting of a braid, knitting, a weave or fabric, exhibits first of all at the start of it which is pulled over the strands of thread or threads, a tubular portion (8) which does not enclose the strands (2) of thread or the threads but stands out sideways from the strands of thread or threads and is separable.

2. An implant as in Claim 1, characterized in that
the end of it sheathed by the sock (7) tapers in conically through different lengths of the individual strands (2) of thread or individual threads.

3. An implant as in Claim 1 or 2, characterized in that
the sock (7) consists of a braid.

## Revendications

1. Implant (1) servant à remplacer des ligaments ou des tendons, constitué d'un nombre de cordes de fils (2) s'étendant dans la direction longitudinale, dont la résistance est renforcée par torsion, tressage ou câblage, ou de fils allongés à monofilament, l'ensemble des cordes de fils (2) ou des fils étant maintenu à une extrémité sur une partie de leur longueur par un bas (7) textile, caractérisé en ce que le bas (7), fait d'un tressage, d'un tricot, d'un tulle ou d'un tissu, comporte, à son début enfilé sur les cordes de fils ou les fils, une partie (8) en forme de tuyau n'enfermant pas les cordes de fils (2) ou les fils, dépassant sur le côté des cordes de fils ou des fils, partie qui peut être coupée.

2. Implant selon la revendication 1, caractérisé en ce que son extrémité enveloppée par le bas (7) se rétrécit coniquement par des longueurs différentes de chacune des cordes de fils (2) ou chacun des fils.

3. Implant selon la revendication 1 ou 2, caractérisé en ce que le bas (7) est constitué d'un tressage.
